# EUROPÄISCHE PATENTSCHRIFT

(11) **EP 1 404 673 B1**
(45) Veröffentlichungstag und Bekanntmachung des Hinweises auf die Patenterteilung: **04.05.2005**
(21) Anmeldenummer: 02727610.4
(22) Anmeldetag: 08.06.2002
(51) Int. Cl.: C07D 451/10, C07D 451/02, C07D 471/08

(54) **TECHNISCHE SYNTHESEVERFAHREN ZUR HERSTELLUNG VON TROPENOL**
TECHNICAL SYNTHESIS METHOD FOR PRODUCING TROPENOL
PROCEDE DE SYNTHESE TECHNIQUE PERMETTANT DE PRODUIRE DU TROPENOL

(30) Priorität: 28.06.2001 DE 10131200
(43) Veröffentlichungstag der Anmeldung: 07.04.2004
(60) Teilanmeldung: 04018550.6
(73) Patentinhaber: Boehringer Ingelheim Pharma GmbH & Co.KG, 55216 Ingelheim am Rhein (DE)
(72) Erfinder: RAPP, Armin, Walter, 55411 Bingen-Dromersheim (DE); SOBOTTA, Rainer, 55218 Ingelheim am Rhein (DE)
(86) Internationale Anmeldenummer: PCT/EP2002/006290
(87) Internationale Veröffentlichungsnummer: WO 2003/002562

(56) Entgegenhaltungen:
- ABERLE N S ET AL: "Parallel modification of tropane alkaloids" TETRAHEDRON LETTERS, ELSEVIER SCIENCE PUBLISHERS, AMSTERDAM, NL, Bd. 42, Nr. 10, 4. März 2001 (2001-03-04), Seiten 1975-1977, XP004316768 ISSN: 0040-4039
- BREMNER J B ET AL: "A Meisenheimer Rearrangement Approach To Bridgehead Hydroxylated Tropane Alkaloid Derivatives" TETRAHEDRON LETTERS, ELSEVIER SCIENCE PUBLISHERS, AMSTERDAM, NL, Bd. 37, Nr. 1, 1996, Seiten 97-100, XP004030485 ISSN: 0040-4039
- DATABASE CA [Online] CHEMICAL ABSTRACTS SERVICE, COLUMBUS, OHIO, US; HAYAKAWA, Y. ET AL: "Carbon-carbon bond formation promoted by transition metal carbonyls. 19. General synthesis of tropane alkaloids via the polybromo ketone-iron carbonyl reaction" retrieved from STN Database accession no. 88:191188 XP002207663 & J. AM. CHEM. SOC. (1978), 100(6), 1786 -91,
- MALPASS J R ET AL: "Synthesis of Epibatidine Homologues: Homoepibatidine and Bis-Homoepibatidine" TETRAHEDRON LETTERS, ELSEVIER SCIENCE PUBLISHERS, AMSTERDAM, NL, Bd. 37, Nr. 22, 1. Mai 1996 (1996-05-01), Seiten 3911-3914, XP004029212 ISSN: 0040-4039

## Beschreibung

Die Erfindung betrifft ein neues, technisch anwendbares Herstellverfahren zur Darstellung von Tropenol, gegebenenfalls in Form seiner Säureadditionssalze.

### Hintergrund der Erfindung

Die Verbindung Tropenol ist aus dem Stand der Technik bekannt und weist die folgende chemische Struktur auf:

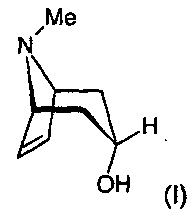

Die Verbindung kann als Ausgangsverbindung zur Darstellung pharmakologisch wertvoller Verbindungen Verwendung finden. Beispielsweise seien in diesem Zusammenhang genannt die Verbindungen Tiotropiumbromid, Ipratropiumbromid oder auch BEA2108. Diese pharmakologisch wertvollen Strukturen sind durch die folgenden chemischen Strukturen gekennzeichnet:

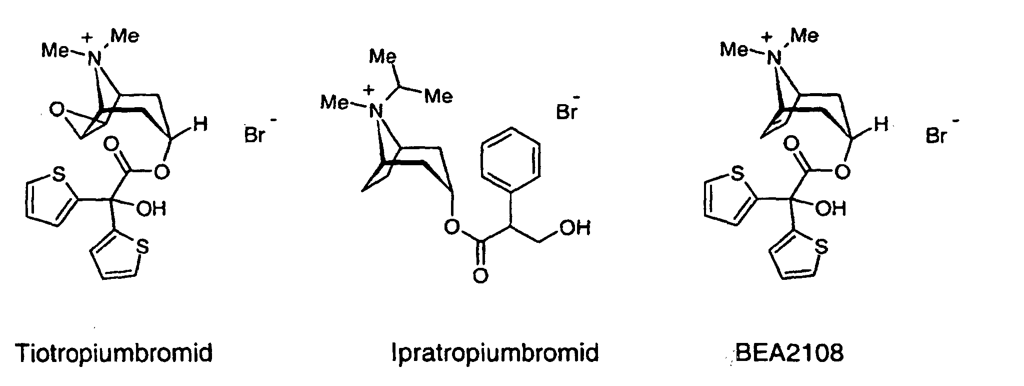

Verfahren zur Herstellung von Tropenol sind aus dem Stand der Technik bekannt. So offenbaren beispielsweise Aberle et al. (Tetrahedron Letters, 42 (2001), 1975-1977) und Bremner et al. (Tetrahedron Letters, 37 (1996), 97-100) synthetische Zugänge zum Tropenol, die jedoch unter anderem dadurch gekennzeichnet sind, dass unter Durchführung zweier Syntheseschritte (reduktive Desoxygenierung und Esterverseifung) unterschiedliche Lösungsmittel, zunächst Ethanol und anschließend das pharmakologisch bedenkliche Methanol bzw. Wasser zum einsatz gelangen.

Aufgrund der hohen Wirksamkeit vorstehend genannter Verbindungen, ist es notwendig, selbige in möglichst hoher Reinheit mittels effizienter Syntheseverfahren zugänglich zu machen. Insbesondere das hohe Reinheitserfordernis, welches generell von therapeutisch zum Einsatz gelangenden Verbindungen erfüllt werden muß, setzt ein möglichst geringes Maß an Verunreinigungen in den Ausgangsverbindungen voraus. Werden bereits als Ausgangsverbindungen Materialien verwendet, die einen relativ hohen Anteil an Verunreinigung enthalten, so gestaltet sich die Aufreinigung des Endprodukts häufig schwierig, da eingangs eingebrachte Verunreinigungen auch in späteren Syntheseschritten häufig nicht mehr ohne weiteres, und wenn dann lediglich unter großen Ausbeuteverlusten abtrennbar sind. Dies ist insbesondere dann der Fall, wenn sich die auftretenden Nebenprodukte bzw. Verunreinigungen von den jeweiligen Hauptprodukten strukturell nur in geringem Maße unterscheiden.

Vor diesem Hintergrund ist es Aufgabe der vorliegenden Erfindung, ein Syntheseverfahren bereitzustellen, welches den technischen Zugang zu Tropenol, vorzugsweise in Form eines seiner Säureadditionssalze, in guter Ausbeute und vor allem hoher Reinheit erlaubt.

### Detaillierte Beschreibung der Erfindung

Die vorstehend definierte Aufgabe wird durch die nachfolgend beschriebene Erfindung gelöst.

Die vorliegende Erfindung betrifft dementsprechend ein technisches Verfahren zur Herstellung von Tropenol der Formel (I) 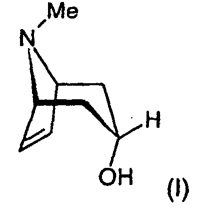 gegebenenfalls in Form seiner Säureadditionssalze, dadurch gekennzeichnet, daß Scopinester der Formel (II) 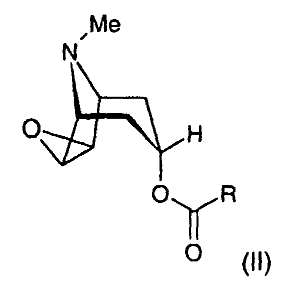 worin
- R: ein Rest ausgewählt aus C₁-C₄-Alkyl und C₁-C₄-alyklen-Phenyl, die jeweils
substituiert sein können durch Hydroxy oder C₁-C₄-Alkoxy, bedeutet, gegebenenfalls in Form ihrer Säureadditionssalze sowie gegebenenfalls in Form ihrer Hydrate in Wasser mittels Zink in Gegenwart aktivierender Metallsalze, bevorzugt aktivierender Eisen- oder Kupfersalze, reduziert und anschließend unter Verwendung geeigneter Basen zu Tropenol der Formel (I) verseift werden.

Unter C₁-C₄-Alkyl werden im Rahmen der vorliegenden Erfindung verzweigte oder unverzweigte Alkylgruppen mit bis zu 4 Kohlenstoffatomen verstanden. Beispielsweise seien genannt Methyl, Ethyl, n-Propyl, iso-Propyl, n-Butyl, sec-Butyl, iso-Butyl und tert-Butyl. Unter C₁-C₄-alkylen-Phenyl wird im Rahmen der vorliegenden Erfindung Phenyl verstanden, welches über eine verzweigte oder unverzweigte Alkylenbrücke mit bis zu 4 Kohlenstoffatomen verknüpft ist, verstanden. Beispielsweise seien genannt Benzyl, Phenyl-2-ethyl-, Phenyl-1-ethyl-, Phenyl-3-propyl-, Phenyl-2-propyl- etc. Sowohl die C₁-C₄-Alkylgruppen als auch die C₁-C₄-alkytenphenyl-gruppen können sofern nicht anders definiert durch einen oder mehrere Hydroxy und/oder C₁-C₄-Alkyloxy-gruppen substituiert sein.

Bevorzugt betrifft die vorliegende Erfindung ein Verfahren zur Herstellung von Tropenol der Formel (I), gegebenenfalls in Form seiner Säureadditionssalze, dadurch gekennzeichnet, daß als Scopinderivat der Formel (II) Scopolamin der Formel (II') 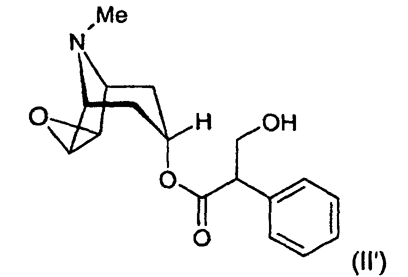 gegebenenfalls in form seiner Säureadditionssalze sowie gegebenenfalls in Form der Hydrate Verwendung findet.

Erfindungsgemäß kann zur Durchführung des Verfahrens zur Herstellung von Tropenol wie folgt vorgegangen werden.
In einem geeigneten Reaktionsgefäß wird Wasser vorzugsweise unter Inertgasatmosphäre, besonders bevorzugt unter Stickstoff vorgelegt. Pro Mol eingesetzte Verbindung der Formel (II) werden erfindungsgemäß zwischen 0,25 und 5, bevorzugt zwischen 0,5 und 3 Litern, besonders bevorzugt zwischen 0,75 und 1,5 Litern Wasser verwendet. In das vorgelegte Wasser wird unter kräftigem Rühren Zink, vorzugsweise in Form von Zinkstaub, besonders bevorzugt von Zinkstaub mit einer mittleren Teilchengröße von <80µm, besonders bevorzugt von <70µm eingebracht. Pro Mol eingesetzte Verbindung der Formel (II) ist der Einsatz von wenigstens 1 Mol Zink erforderlich. Erfindungsgemäß bevorzugt wird Zink allerdings im Überschuß eingesetzt. Vorzugsweise werden pro Mol eingesetzte Verbindung der Formel (II) 1,2 bis 3,5 Mol, besonders bevorzugt 1,5 bis 3,0 Mol Zink verwendet. In einer besonders bevorzugten Ausführungsform des erfindungsgemäßen Verfahrens werden 1,8 bis 2,5 Mol Zink pro Mol eingesetzte Verbindung der Formel (II) verwendet. Nach Zugabe des Zinks kann es förderlich sein eine Aktivierung desselben vorzunehmen. Diese kann durch Zugabe von HI, HBr oder beispielsweise HCl erfolgen. Bevorzugt wird als Aktivierungsagens HI, vorzugsweise in Form wässeriger Lösungen, besonders bevorzugt in Form konzentrierter wässeriger Lösungen durchgeführt. Pro Mol eingesetzte Verbindung der Formel (II) kann beispielsweise die Zugabe von 0,05 - 0,25 Mol, bevorzugt von 0,08 bis 0,2 Mol Aktivierungsagens förderlich sein. Gegebenenfalls kann es hilfreich sein, vor Zugabe des Aktivierungsreagenzien die Temperatur der vorgelegten Mischung zu erhöhen. Vorzugsweise wird dann auf eine Temperatur von über 50°C, vorzugsweise 55-90°C, besonders bevorzugt 60-80°C erwärmt. Anschließend erfolgt die Zugabe der Metallsalze zur gegebenenfalls mittels eines der vorstehend genannten Agenzien aktivierten Suspension von Zink in Wasser. Als im Rahmen der vorliegenden Erfindung einsetzbare Metallsalze sind Salze des Eisens (vorzugsweise Fe-(III)-Salze) oder des Kupfers (vorzugsweise Cu-(II)-Salze), vorzugsweise deren Halogenide zu nennen. Als Eisensalz gelangt bevorzugt FeCl₃ zur Anwendung. Besonders bevorzugt werden im Rahmen des erfindungsgemäßen Verfahrens allerdings Cu-(II)-Salze verwendet, die ausgewählt sind aus der Gruppe CuCl₂, CuI₂, CuBr₂ und CuBr₂-Dimethylsulfidkomplex, wobei CuBr₂ erfindungsgemäß eine besondere Bedeutung zu kommt. Pro Mol eingesetzte Verbindung der Formel (II) werden erfindungsgemäß stets substöchiometrische Mengen des Metallsalzes, vorzugsweise 0,01 bis < 1 Mol Metallsalz zugesetzt. Bevorzugt finden pro Mol eingesetzte Ausgangsverbindung (II) 0,05 bis 0,5 Mol, besonders bevorzugt 0,075 bis 0,2 Mol Metallsalz Verwendung. Das Metallsalz kann in Substanz oder in gelöster Form der Zinksuspension zugeführt werden. Erfindungsgemäß bevorzugt wird das Metallsalz allerdings in Wasser aufgenommen und dann in gelöster oder suspendierter Form der Zinksuspension zugeführt. Pro Mol eingesetztes Metallsalz werden erfindungsgemäß 0,5 bis 1,5 Liter, vorzugsweise 0,6 bis 1,0 Liter Wasser zur Herstellung der Metallsalz-Lösung oder -Suspension verwendet. Diese Lösung oder Suspension wird dann der vorgelegten Zink-Mischung unter Rühren zugeführt.

Zu der nach vorstehend beschriebener Vorgehensweise erhältlichen Zink Mischung wird nun die Verbindung der Formel (II) gegeben. Die Zugabe kann gegebenenfalls in Form der Säureadditionssalze von (II) erfolgen. Diese Säureadditionssalze sind erfindungsgemäß vorzugsweise ausgewählt aus der Gruppe bestehend aus Hydrochlorid, Hydrobromid, Hydrogenphosphat, Hydrogensulfat, Tetrafluoroborat und Hexafluorophosphat, wobei die Hydrochloride oder Hydrobromide besonders bevorzugt sind. Eine Bezugnahme auf Säureadditionssalze der Verbindung der Formel (II) schließt eine Bezugnahme auf deren gegebenenfalls existierende Hydrate mit ein. Erfolgt die direkte Zugabe der vorstehend genannten Säureadditionssalze, so können diese der vorgelegten Zink-Mischung in Substanz oder in gelöster Form zugeführt werden. Werden die Säureadditionssalze in gelöster Form zugegeben, empfiehlt sich die Aufnahme der Säureadditionssalze der Verbindungen der Formel (II) in Wasser.
Erfindungsgemäß bevorzugt werden pro Mol eingesetztes Säureadditionssalz der Formel (II) 0,5 bis 1,5 Liter, vorzugsweise 0,6 bis 1,0 Liter Wasser verwendet.

Alternativ dazu ist es möglich, die Verbindungen der Formel (II) in Form ihrer freien Basen in einer separaten Versuchsanordnung zunächst in Wasser mittels der entsprechenden Säuren in die gelösten Säureadditionssalze zu überführen, und diese Lösung anschließend der vorgelegten Zink-Mischung zuzuführen. Erfindungsgemäß bevorzugt werden pro Mol eingesetzte freie Base der Formel (II) 0,5 bis 1,5 Liter, vorzugsweise 0,6 bis 1,0 Liter Wasser verwendet. Die dadurch erhaltene Suspension wird anschließend mit der zur Bildung des Säureadditionssalzes Hydrochlorid, Hydrobromid, Hydrogenphosphat, Hydrogensulfat, Tetrafluoroborat oder Hexafluorophosphat erforderlichen entsprechenden Säure versetzt. Pro Mol eingesetzte freie Base der Formel (II) sind wenigstens 1 Mol der jeweiligen Säure einzusetzen. Allerdings ist es im Rahmen der erfindungsgemäßen Verfahren durchaus möglich die Säure im Überschuß (d.h. 1,1 bis etwa 2 Mol pro Mol Base (II)) zu verwenden. Erfindungsgemäß bevorzugt werden die Hydrochloride oder Hydrobromide der Verbindungen (II) verwendet. Die Zugabe der Salzsäure kann entweder in Form wässeriger Lösungen oder gasförmig erfolgen, wobei die Zugabe wässeriger Lösungen bevorzugt ist. Vorzugsweise wird konzentrierte Salzsäure (36%-ig) in Wasser gelöst zugegeben. Wird, was erfindungsgemäß besonders bevorzugt ist, Bromwasserstoffsäure verwendet, so kann deren Zugabe ebenfalls entweder in Form wässeriger Lösungen oder gasförmig erfolgen, wobei die Zugabe wässeriger Lösungen bevorzugt ist. Vorzugsweise wird konzentrierte Bromwasserstoffsäure (62%-ig) in Wasser gelöst zugegeben. Durch die Zugabe einer der vorstehend genannten Säuren zur Suspension der freien Base der Formel (II) im jeweiligen Lösemittel wird ein pH von 3,5 bis 5,5, vorzugsweise von 4,5 bis 5 eingestellt.

Die vorstehend beschriebenen und gegebenenfalls nach unterschiedlichen Wegen zugänglichen Lösung der Säureadditionssalze der Formel (II) wird anschließend der vorgelegten Zinksuspension zugeführt. Die Zugabe kann beispielhaft aber nicht notwendigerweise bei erhöhter Temperatur erfolgen. Eine solche erhöhte Temperatur bietet sich allerdings vor allem dann an, wenn die Mischung bereits vor Zugabe des Aktivierungsreagenzes erwärmt worden ist. Erfolgt die Zugabe bei erhöhter Temperatur, so bieten sich erfindungsgemäß Temperaturen von über 50°C, vorzugsweise 55-90°C, besonders bevorzugt 60-80°C an.

Nach beendeter Zugabe wird die Reaktionsmischung in einem Temperaturbereich von 50 bis 100°C, vorzugsweise von 60 bis 95°C, besonders bevorzugt bei etwa 70 bis 85°C gerührt. Es wird solange bei konstanter Temperatur gerührt, bis der Umsatz vollständig ist (0,5 bis 4 Stunden). Die Kontrolle des Fortgangs der Reaktion kann beispielsweise mittels Dünnschichtchromatographie erfolgen.

Nach vollständigem Umsatz wird die Reaktionsmischung zur Verseifung der Esterfunktion mit einer geeigneten Base versetzt. Als Base kommen bevorzugt anorganische Basen ausgewählt aus der Gruppe der Alkali- oder Erdalkalicarbonate, Alkali- oder Erdalkalialkoholate und Alkali- oder Erdalkalihydroxyide in Betracht. Besonders bevorzugt sind dabei die Hydroxide des Lithiums, Natriums, Kaliums und Calziums besonders bevorzugt des Natriums oder Kalziums. Erfindungsgemäß besonders bevorzugt wird Natriumhydroxid als Base verwendet. Vorstehend genannte Basen können in Reinform oder, besonders bevorzugt, in Form wässeriger konzentrierter Lösungen zum Einsatz gelangen. Wird beispielsweise die erfindungsgemäß besonders bevorzugte Base Natriumhydroxid verwendet, so erfolgt deren Zugabe vorzugsweise in Form wässeriger Lösungen mit einer Konzentration von wenigstens 40 Gew%. Pro Mol ursprünglich eingesetzte Verbindung der Formel (II) ist der Einsatz wenigstens stöchiometrischer Mengen an Base erforderlich. Allerdings ist auch die Verwendung der Base im Überschuß möglich. Entweder erfolgt bereits die Zugabe der Base bei einer Temperatur in einem Bereich von 0 bis 50°C, vorzugsweise von 10 bis 40°C, besonders bevorzugt bei etwa 20 bis 30°C oder vorstehende Temperatur wird umgehend nach Zugabe der Base eingestellt. Es wird solange bei dieser Temperatur gerührt, bis der Umsatz vollständig ist (je nach Ansatzgröße 12 bis 24 Stunden). Bei kleineren Ansatzgrößen (z.B. Kilogrammaßstab) kann die Verseifung auch bei erhöhter Temperatur (50-100°C, vorzugsweise 55-90°C, besonders bevorzugt etwa 60 bis 80°C) erfolgen. Dadurch kann sich die Reaktionszeit auf etwa 15 Minuten bis 10 Stunden, vorzugsweise 0,5-3 Stunden verkürzen. Die Kontrolle des Fortgangs der Reaktion kann beispielsweise mittels Dünnschichtchromatographie erfolgen.

Nach vollständigem Umsatz wird die Reaktionsmischung unter Rühren auf eine Temperatur im Bereich von 0 und 50°C, vorzugsweise 15 bis 45 °C gebracht und mittels Filtration von den Zinksalzen befreit. Der Filterrückstand kann gegebenenfalls durch das für die Reaktion verwendete Lösemittel gewaschen werden. Das Filtrat wird zur Extraktion mit einem organischen Lösemittel, welches nicht oder nur in geringem Maße mit Wasser mischbar ist, versetzt. Vorzugsweise wird ein organisches Lösemittel ausgewählt aus der Gruppe bestehend aus Methyl-tertbutylether, Dichlormethan, Chloroform, vorzugsweise Dichlormethan verwendet. Pro Mol eingesetzte Verbindung der Formel (II) werden erfindungsgemäß zwischen 0,5 und 5, bevorzugt zwischen 0,75 und 4 Litern organisches Lösemittel für die Extraktion verwendet. Die Extraktion wird erfindungsgemäß zwischen 3 und 8, bevorzugt 4 bis 6 mal durchgeführt. Nach beendeter Extraktion werden die organischen Phasen vereint und das organische Lösemittel im Vakuum abdestilliert.

Das verbleibende Rohprodukt wird in einem organischen Lösemittel ausgewählt aus der Gruppe bestehend aus Methanol, Ethanol, Isopropanol, vorzugsweise Isopropanol aufgenommen. Pro Mol ursprünglich eingesetzte Verbindung der Formel (II) werden erfindungsgemäß zwischen 0,1 und 2,0 Litern, vorzugsweise zwischen 0,3 und 1,0 Litern dies vorstehend genannten Lösemittels verwendet. Die erhaltene Lösung wird von ausgefallenem Feststoff (Metallsalze der Säure RCOOH, wobei R die eingangs genannten Bedeutungen aufweisen kann) mittels Filtration abgetrennt. Das Filtrat enthält Tropenol der Formel (I) in Form seiner freien Base. Sofern die freie Base in die nächste Umsetzung eingesetzt werden soll, erfolgt an dieser Stelle das Abdestillieren des Lösemittels unter Vakuum. Die verbleibende freie Base ist dann, ohne weitere Reinigung, in die nächsten Syntheseschritte einsetzbar. Erfindungsgemäß bevorzugt wird die freie Base des Tropenols aber in eines der Säureadditionssalze überführt. Als Säureadditionssalze des Tropenols werden im Rahmen der vorliegenden Erfindung die Salze verstanden, die ausgewählt sind aus der Gruppe bestehend aus Hydrochlorid, Hydrobromid, Hydrogenphosphat, Hydrogensulfat, Tetrafluoroborat oder Hexafluorophosphat. Besonders bevorzugt sind dabei die Salze Hydrobromid und Hydrochlorid, wobei dem Tropenolhydrochlorid erfindungsgemäß eine besondere Bedeutung zu kommt. Zur Darstellung der Säureadditionssalze wird das Filtrat auf eine Temperatur im Bereich von -10°C bis 20°C, vorzugsweise im Bereich von -5°C und 15°C gekühlt. Die dadurch erhaltene Suspension wird anschließend mit der zur Bildung des Säureadditionssalzes Hydrochlorid, Hydrobromid, Hydrogenphosphat, Hydrogensulfat, Tetrafluoroborat oder Hexafluorophosphat erforderlichen entsprechenden Säure versetzt. Pro Mol ursprünglich eingesetzte freie Base der Formel (II) sind wenigstens 1 Mol der jeweiligen Säure einzusetzen. Gegebenenfalls ist es im Rahmen der erfindungsgemäßen Verfahren möglich, die Säure im Überschuß (d.h. 1,1 bis etwa 2-3 Mol pro Mol ursprünglich eingesetzte Base (II)) zu verwenden. Erfindungsgemäß bevorzugt wird das Hydrochlorid des Tropenols hergestellt. Die Zugabe der dafür erforderlichen Salzsäure kann entweder in Form von Lösungen oder gasförmig erfolgen. Vorzugsweise wird Chlorwasserstoff in gasförmiger Form in einem separaten Reaktionsgefäß in eines der vorstehend genannten Lösemittel bis zur Sättigung eingereicht. Besonders bevorzugt wird zur Darstellung dieser HCl-Lösung das Lösemittel verwendet, welches zur Herstellung des Tropenol-filtrats Verwendung findet. Die Zugabe einer der vorstehend genannten Säuren zur Lösung der freien Base des Tropenols (I) erfolgt, bis ein pH von 1,5 bis 6,5, vorzugsweise von 2 bis 6 erreicht ist. Nach beendeter Zugabe der Säure kann bei konstanter Temperatur gegebenenfalls noch 0,5 bis 2 Stunden nachgerührt werden. Abschließend wird das ausgefallene Säureadditionssalz des Tropenols abgetrennt und gegebenenfalls mit einem Lösemittel ausgewählt aus der Gruppe bestehend aus Aceton, Methylisobutylketon und Methylethylketon, vorzugsweise Aceton gewaschen und im Vakuum getrocknet.

Wie einleitend formuliert stellt Tropenol, welches gemäß dem erfindungsgemäßen Herstellverfahren zugänglich ist, eine wertvolle Ausgangsverbindung zur Darstellung therapeutisch wirksamer Verbindungen wie beispielsweise Tiotropiumbromid, lpratropiumbromid oder BEA2108 dar. Aufgrund der hohen Reinheit, in der Tropenol gemäß der vorliegenden Erfindung zugänglich wird, ist es möglich die vorstehend genannten Wirkstoffe in den für die pharmazeutische Anwendung notwendigen Spezifikationen bereit zu stellen.

Zur Darstellung von Tiotropiumbromid ausgehend von Tropenol kann wie in Schema 1 dargestellt, vorgegangen werden.

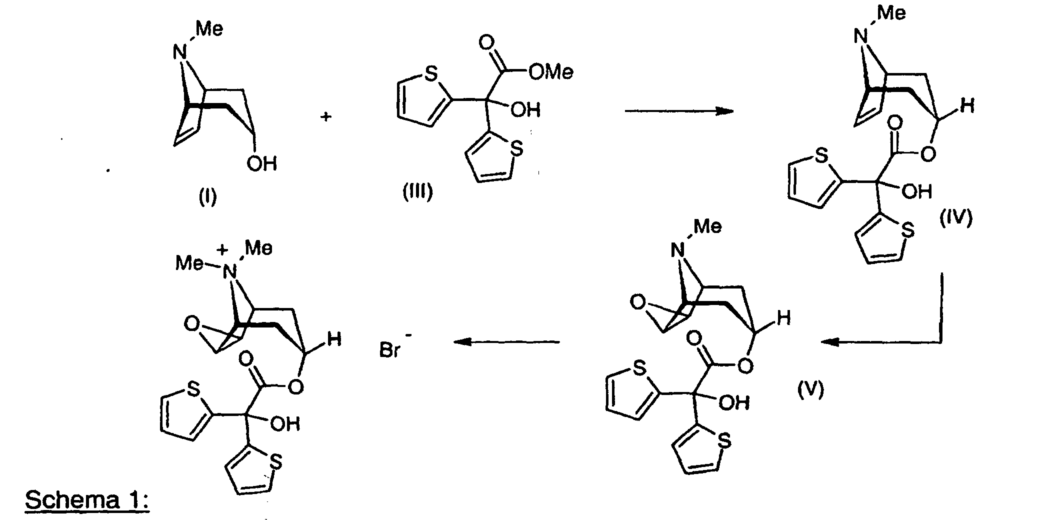

Ausgehend von gemäß dem erfindungsgemäß erhältlichen Tropenol (I) erfolgt durch Umsetzung mit Di-(2-thienyl)-glykolsäurederivaten (III) zunächst die Bildung des Di-(2-thienyl)-glykolsäure-tropenotesters (IV). Dieser wird durch Oxidation der olefinischen Doppelbindung in den entsprechenden Scopinester (V) überführt, aus dem Tiotropiumbromid durch Umsetzung mit Methylbromid erhältlich wird.

Die nachfolgenden Beispiele dienen der Illustration exemplarisch durchgeführter Syntheseverfahren zur Herstellung von Tiotropiumbromid. Sie sind lediglich als mögliche, exemplarisch dargestellte Vorgehensweisen zu verstehen, ohne die Erfindung auf deren Inhalt zu beschränken.

### Beispiel 1:

### Darstellung von Tropenol (I) in Form seines Hydrochlorids (Kilogrammmaßstab)

In einem mit Stickstoff-gespülten Reaktor werden 3 Liter Wasser vorgelegt und unter kräftigem Rühren 390 g Zinkstaub (<63µm) und zur Aktivierung 66 ml 57%-ige, wässerige Iodwasserstofflösung zugesetzt. Diese Mischung wird bei Raumtemperatur etwa 5 Minuten gerührt. Anschließend erfolgt die langsame Zugabe von 67,2 g Cu(II)-Bromid, gelöst in 260 ml Wasser. Zu dieser Mischung wird langsam eine Lösung von 910,2 g Scopoiamin-Base, aufgenommen in etwa 2,6 Liter Wasser gegeben und mittels 227 ml 62%-iger, wässeriger Bromwasserstofflösung auf einen pH von 4,5-5 eingestellt, gegeben. Nach beendeter Zugabe wird auf eine Temperatur von 75-80°C erwärmt und etwa 2 Stunden bei dieser Temperatur gerührt. Nach vollständiger Umsetzung (DC-Kontrolle) wird auf etwa 65°C abgekühlt. Es werden 480 ml einer 45%-igen wässerigen Natronlauge zugegeben und bei einer Temperatur von 65-70°C bis zur vollständigen Verseifung gerührt (etwa 1 Stunde). Nach Abkühlen auf etwa 40°C werden die Zn-Salze abfilitriert und mit etwa 200 ml Wasser nachgewaschen. Das Filtrat wird mittels Dichlormethan mehrfach extrahiert (3-5 mal, jeweils 2-4 Liter Dichlormethan), die organischen Phasen vereint und das Lösemittel unter reduziertem Druck abdestilliert. Der verbleibende Rückstand (371 g Rohprodukt) wird in 1,5 Litern Isopropanol aufgenommen und der ausgefallene Feststoff (Tropasäure-Metallsalz) abfiltriert. Das Filtrat wird auf -10°C bis 10°C gekühlt und langsam und unter kräftigem Rühren mit 120 g HCl, gelöst in 780 ml Isopropanol, versetzt. Hierbei wird ein pH von 2,5 - 4 eingestellt. Nach beendeter Zugabe wird bei etwa -5 °C noch eine weitere Stunde gerührt. Die Suspension wird abschließend filtriert, der Filterrückstand mittels etwa 600 ml Aceton nachgewaschen und abschließend im Vakuum bei etwa 60°C getrocknet.
Ausbeute: 408,1 g Tropenolhydrochlorid (77,4% bezogen auf eingesetztes Scopolamin)

### Beispiel 2:

### Darstellung von Tropenol (I) in Form seines Hydrochlorids (großtechnisch)

In einem mit Stickstoff-gespülten Reaktor werden 130 Liter Wasser vorgelegt und unter kräftigem Rühren 21,5 kg Zinkstaub (<63µm) zugegeben. Diese Mischung wird auf eine Temperatur von 65-75°C erwärmt. Zu dieser Mischung werden 6,2 kg 57%ige wässerige Jodwasserstoffsäure gegeben. Anschließend erfolgt die Zugabe einer Lösung von 3,7 kg Cu(II)-Bromid in 20-25 Liter Wasser. Es wird gegebenenfalls bis zu 5 Minuten nachgerührt und anschließend eine Lösung von 65,8 kg Scopolaminhydrobromid-trihydrat in 140-145 Liter Wasser zugegeben. Die erhaltene Mischung wird auf 75-85°C erwärmt und 2-2,5 Stunden gerührt. Nach vollständigem Umsatz (DC-Kontrolle) werden 35,5 kg einer 45%-igen wässerigen Natronlauge zugegeben. Es wird auf eine Temperatur von 20-30°C gebracht und der Ansatz weitere 20-24 Stunden gerührt. Nach vollständigem Umsatz (DC-Kontrolle) wird der gesammte Apparateinhalt filtriert und der verbleibende Rückstand mit etwa 30 Liter Wasser nachgewaschen. Das Filtrat wird bei konstanter Temperatur mit 75 kg Natriumchlorid versetzt. Zur Extraktion erfolgt die Zugabe von 150 Liter Dichlormethan. Die organische Phase wird abgetrennt und die wässerige Phase weitere 4 mal mit der gleichen Menge Dichlormethan extrahiert. Die vereinten organischen Phasen werden destillativ vom Lösemittel befreit. Zum verbleibenden Rückstand werden etwa 100 Liter Isopropanol gegeben und auf eine Temperatur von 0-10°C eingestellt. Sodann erfolgt die Zugabe einer Lösung von 5,5 kg Chlorwasserstoff in 38 Litern Isopropanol bis ein pH von etwa 2,5-5,5 eingestellt ist. Das ausgefallene Tropenolhydrochlorid wird abgetrennt und mit 30 Litern Aceton nachgewaschen. Nach Trocknung werden 21,3 kg Produkt (Ausbeute 81% bezogen auf eingesetztes Scopolaminhydrobromid) erhalten.

### Beispiel 3: Darstellung von Tiotropiumbromid

### a) Darstellung des Tropenolesters (IV)

Zu 10,9 kg Tropenolhydrochlorid (erhältlich nach Beispiel 1) in Toluol (95 L) wird bei 25°C Ammoniak (1,8 kg) eingeleitet. Die erhaltene Suspension wird bei konstanter Temperatur etwa 1 h gerührt. Anschließend wird entstandenes Ammoniumhydrochlorid abfiltriert und mit Toluol (26 L) nachgespült. Bei etwa 50°C Manteltemperatur wird ein Teil des Toluols (ca 60 L) im Vakuum abdestilliert. Nach Abkühlen auf etwa 25°C werden 15,8 kg Di-(2-thienyl)glykolsäuremethylester zugeben und die erhaltene Mischung zum Lösen auf 50°C erwärmt. In einem weiteren Apparat wird Toluol (40 L) vorgelegt und in diese bei etwa 25°C Natriumhydrid (2,7 kg) eingetragen. In diese Lösung wird innerhalb von 1 h die zuvor generierte Lösung aus Tropenol und Glykolsäuremethylester bei 30°C gegeben. Nach beendeter Zugabe wird bei reduziertem Druck etwa 7 Stunden unter Rühren auf 75°C erhitzt. Das sich bildende Methanol und wird dabei abdestilliert. Die verbleibende Mischung wird abgekühlt und in eine Mischung von Wasser (958 L) und 36 %iger Salzsäure (13,2 kg) gegeben. Die wäßrige Phase wird anschließend abgetrennt und mit Methylenchlorid (56 L) waschen. Nach erneuter Methylenchloridzugabe (198 L) wird das so erhaltene Gemisch mit vorbereiteter Sodalösung (9,6 kg Soda in 45 L Wasser) auf pH = 9 gestellt. Die Methylenchlorid-Phase wird abgetrennt und die wässerige Phase mit Methylenchlorid (262 L) ausgerührt. Die Methylenchlorid-Phase werden bei 65°C bis auf den Rückstand eingeengt. Den Rückstand wird in Toluol (166 L) aufgenommen und auf 95°C erhitzt. Die toluolische Lösung wird auf 0°C abgekühlt. Die erhaltenen Kristalle werden abgetrennt, mit Toluol (33 L) gewaschen und bei etwa 50°C für max. 24 Std. im Stickstoffstrom getrocknet.
Ausbeute: 18,6 kg (83%);Schmp.: ca. 160°C (bestimmt über DSC bei Heizrate von 10K/min);

### b) Darstellung des Scopinesters (V)

In einem geeigneten Reaktionsapparat werden 260 L DMF vorgelegt und auf 50°C erwärmt. Anschließend werden 16,2 kg Tropenolester (IV) zugegeben und es wird gerührt, bis eine klare Lösung erhalten worden ist. Nach Abkühlen auf 40°C werden nacheinander und portionsweise Wasserstoffperoxid-Harnstoff-Komplex (10,2 kg), Wasser (13 L) und Vanadium-(V)-oxid (0,7 kg) eintragen und der Apparateinhalt auf etwa 50°C erhitzt. Nach 2 - 3 h Rühren bei konstanter Temperatur wird etwa 20°C abgekühlt. Das erhaltene Reaktionsgemisch wird mit Salzsäure (36 %ig) auf etwa pH 4,0 eingestellt. Vorbereitete Natriumbisulfit-Lösung (2,4 kg in 24 L Wasser) wird zugegeben. Bei einer Innentemperatur von 35°C wird das Lösemittel im Vakuum teilweise abdestilliert (etwa 210 L). Es wird wiederum auf etwa 20°C abgekühlt und mit Clarcel (3,2 kg) versetzt. Mit verdünnter Salzsäure (36%-ig, 0,8 kg in etwa 440 L Wasser) wird auf einen pH von etwa 2,0 eingestellt. Die erhaltene Lösung wird filtriert und mit Methylenchlorid (58 L) extrahiert. Die Methylenchloridphase wird verworfen. Zur wäßrigen Phase wird erneut Methylenchlorid (130 L) gegeben und mit einer vorbereiteten Soda-Lösung (11,0 kg in 51 L Wasser) wird ein pH von etwa 10,0 eingestellt. Die Methylenchlorid-Phase wird abgetrennt und die wäßrige Phase mit Methylenchlorid (136 L) extrahiert. Im schwachen Vakuum (600 - 700 mbar) wird bei 40°C von den vereinigten Methylenchloridphasen Methylenchlorid (etwa 175 L) abdestilliert. Der Apparateinhalt wird auf 20°C abgekühlt, Acetylchlorid (etwa 0,5 kg) wird zugesetzt und es wird für etwa 40 Minunten bei 20°C gerührt. Die Reaktionslösung wird in einen zweiten Apparat überführt. Mit einer vorbereiteten Salzsäure-Lösung (4,7 kg Salzsäure 36 %ig in 460 L Wasser) wird bei 20°C ein pH von 2,0 eingestellt. Die Methylenchlorid-Phase wird abgetrennt und entsorgt. Die wäßrige Phase wird mit Methylenchlorid (39 L) gewaschen. Dann wird Methylenchlorid (130 L) zugesetzt und mit einer vorbereiteten Soda-Lösung (7,8 kg Soda in 38 L Wasser) bei 20°C ein pH von 10,0 eingestellt. Nach 15 Min. Rühren wird die organische Phase abgetrennt und die wäßrige Phase zweimal mit Methylenchlorid (97 L und 65 L) gewaschen. Die Methylenchlorid-Phasen werden vereinigt und im schwachen Vakuum ein Teil des Methylenchlorids (90 L) bei einer Temperatur von 30 - 40°C abdestilliert. Anschließend wird Dimethylformamid (114 kg) zugesetzt und unter Vakuum bei 40°C das restliche Methylenchlorid abdestilliert. Der Apparateinhalt wird auf 20°C abgekühlt.

### c) Darstellung des Tiotropiumbromids

Zur nach vorhergehender Vorschrift erhältlichen Scopinester-Lösung wird bei 20°C Methylbromid (5,1 kg) eingeleitet. Der Anlageninhalt wird bei 30°C für etwa 2,5 Tage gerührt. Bei 50°C werden im Vakuum 70 L DMF abdestilliert. Die Lösung wird in einen kleineren Apparat überführt. Es wird mit DMF (10 L) nachgespült.. Bei 50°C wird im Vakuum weiteres DMF abdestilliert bis eine Gesamtdestillatmenge von etwa 100 L erreicht ist. Es wird auf 15°C abgekühlt und bei dieser Temperatur 2 h nachgerührt. Das Produkt wird mittels Nutschentrocker isoliert, mit 15°C kaltem DMF (10 L) und 15°C kaltem Aceton (25 L) gewaschen. Es wird bei max. 50°C für max. 36 Std. im Stickstoffstrom getrocknet. Ausbeute: 13,2 kg (88 %);
Schmp.: 200-230°C (abh. von Reinheitsgrad des Rohprodukts);

Das so erhaltene Rohprodukt (10,3 kg) wird in Methanol (66 L) eingetragen. Das Gemisch wird zum Lösen zum Rückfluß erhitzt. Die Lösung wird auf 7°C abgekühlt und 1,5 h bei dieser Temperatur nachgerührt. Das Produkt wird mittels Nutschentrockner isoliert, mit 7°C kaltem Methanol (11 L) gewaschen und max. 36 h bei etwa 50°C im Stickstoffstrom getrocknet. Ausbeute: 9,9 kg (96 %);
Schmp.: 228°C (bestimmt über DSC bei Heizrate von 10K/min).

Gegebenenfalls kann das so erhaltene Produkt in das kristalline Monohydrat desTiotropiumbromids überführt werden. Hierzu kann wie folgt vorgegangen werden. In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei etwa 25°C über etwa 2 Stunden im Stickstoffstrom getrocknet. Ausbeute : 13,4 kg Tiotropiumbromid-monohydrat (86 % d. Th.)
Schmp.: 230°C (bestimmt über DSC bei Heizrate von 10K/min).

## Patentansprüche

1. Verfahren zur Herstellung von Tropenol (I) 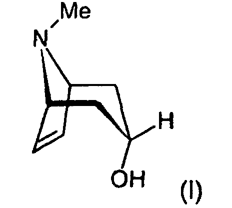 gegebenenfalls in Form seiner Säureadditionssalze, **dadurch gekennzeichnet, daß** Scopinester der Formel (II) 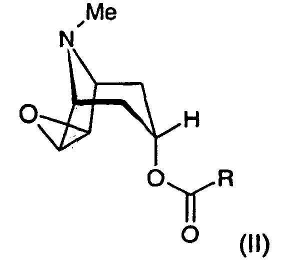 worin
R ein Rest ausgewählt aus C₁-C₄-Alkyl und C₁-C₄-alyklen-Phenyl, die jeweils substituiert sein können durch Hydroxy oder C₁-C₄-Alkoxy, bedeutet,
gegebenenfalls in Form ihrer Säureadditionssalze sowie gegebenenfalls in Form ihrer Hydrate, in Wasser mittels Zink in Gegenwart aktivierender Metallsalze, bevorzugt aktivierender Eisen- oder Kupfersatze, reduziert und anschließend unter Verwendung geeigneter Basen zu Tropenol der Formel (I) verseift werden.

2. Verfahren nach Anspruch 1, **dadurch gekennzeichnet, daß** als Scopinderivat der Formel (II) Scopolamin der Formel (II') 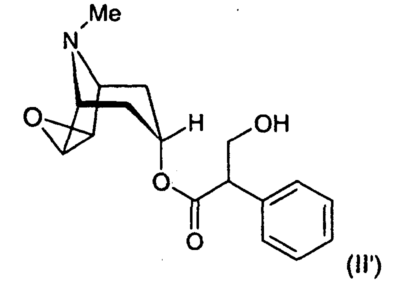 gegebenenfalls in form seiner Säureadditionssalze sowie gegebenenfalls in Form der Hydrate Verwendung findet.

3. Verfahren nach Anspruch 1 oder 2, **dadurch gekennzeichnet, daß**
in einem ersten Schritt in Wasser Zink vorgelegt wird,
in einem zweiten Schritt, gegebenenfalls nach Aktivierung mit einem geeigneten Aktivierungsreagenz, die Zugabe des Metallsalzes erfolgt,
in einem dritten Schritt die Verbindung der Formel (II) gegebenenfalls in Form eines ihrer Säureadditionssalze und/oder Hydrate zugesetzt wird,
in einem vierten Schritt die Esterfunktion mittels einer geeigneten Base verseift wird und schließlich die Verbindung der Formel (I), gegebenenfalls in Form eines ihrer Säureadditionssalze isoliert wird.

4. Verfahren nach einem der Ansprüche 1, 2 oder 3, **dadurch gekennzeichnet, daß** als Metallsalz Salze des Fe-(III) oder des Cu-(II), vorzugsweise deren Halogenide zur Anwendung gelangen.

5. Verfahren nach Anspruch 4, **dadurch gekennzeichnet daß** die Salze ausgewählt sind aus der Gruppe FeCl₃, CuCl₂, Cul₂, CuBr₂ und CuBr₂-Dimethylsulfidkomplex, vorzugsweise CuBr₂.

## Claims

1. Process for preparing tropenol (I) 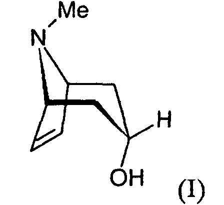 optionally in the form of the acid addition salts thereof, **characterised in that** scopine esters of formula (II) 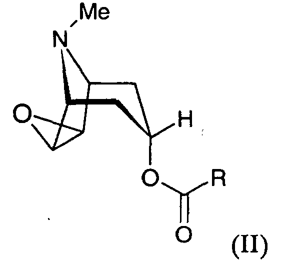 wherein
R denotes a group selected from C₁-C₄-alkyl and C₁-C₄-alkylene-phenyl which may be substituted in each case by hydroxy or C₁-C₄-alkoxy,
are reduced, optionally in the form of the acid addition salts thereof as well as optionally in the form of the hydrates thereof, in water using zinc in the presence of activating metal salts, preferably activating iron or copper salts, and are subsequently saponified using suitable bases to obtain tropenol of formula (I).

2. Process according to claim 1, **characterised in that** scopolamine of formula (II') 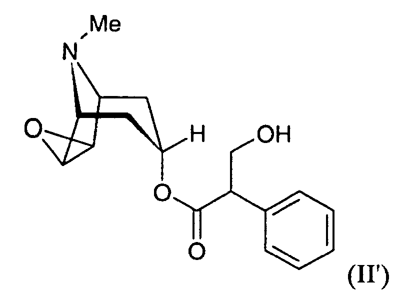 is used as the scopine derivative of formula (II), optionally in the form of the acid addition salts thereof as well as optionally in the form of the hydrates thereof.

3. Process according to claim 1 or 2, **characterised in that**
in a first step zinc is placed in water,
in a second step, optionally after activation with a suitable activating reagent, the metal salt is added,
in a third step the compound of formula (II) is added, optionally in the form of one of the acid addition salts and/or hydrates thereof,
in a fourth step the ester function is saponified using a suitable base and finally the compound of formula (I) is isolated, optionally in the form of one of the acid addition salts thereof.

4. Process according to one of claims 1, 2 or 3, **characterised in that** salts of Fe-(III) or Cu-(II), preferably the halides thereof, are used as the metal salt.

5. Process according to claim 4, **characterised in that** the salts are selected from among FeCl₃, CuCl₂, CuI₂, CuBr₂ and CuBr₂-dimethylsulphide complex, preferably CuBr₂.

## Revendications

1. Procédé de préparation du tropénol (I) 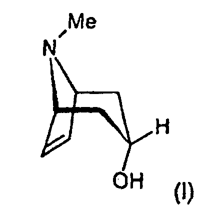 éventuellement sous forme de ses sels d'addition d'acide, **caractérisé en ce qu'**un ester de scopine de formule (II) 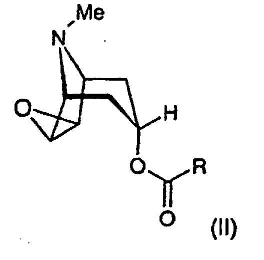 où
R représente un groupement choisi parmi C₁-C₄-alkyle et C₁-C₄-alkylène-phényle, qui peuvent être substitués chacun par hydroxyle ou C₁-C₄-alcoxy,
éventuellement sous forme de ses sels d'addition d'acide ainsi que éventuellement sous forme de ses hydrates, est réduit dans l'eau avec du zinc en présence de sels métalliques activants, de préférence de sels de fer ou de cuivre activants, puis saponifié en le tropénol de formule (I) au moyen de bases appropriées.

2. Procédé selon la revendication 1 **caractérisé en ce que** la scopolamine de formule (II') 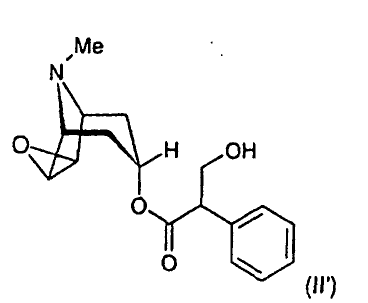 éventuellement sous forme de ses sels d'addition d'acide ainsi que éventuellement sous forme des hydrates est utilisée comme dérivé de scopine de formule (II).

3. Procédé selon la revendication 1 ou 2 **caractérisé en ce que**, dans une première étape, du zinc est disposé au préalable dans l'eau, dans une deuxième étape, éventuellement après activation avec un réactif d'activation approprié, a lieu l'addition du sel métallique, dans une troisième étape le composé de formule (II) éventuellement sous forme de l'un de ses sels d'addition d'acide et/ou hydrates est ajouté, dans une quatrième étape la fonction ester est saponifiée au moyen d'une base appropriée et enfin le composé de formule (I) est isolé, éventuellement sous forme de l'un de ses sels d'addition d'acide.

4. Procédé selon l'une des revendications 1, 2 ou 3 **caractérisé en ce que** des sels de Fe (III) ou de Cu (II), de préférence leurs halogénures, sont utilisés comme sel métallique.

5. Procédé selon la revendication 4 **caractérisé en ce que** les sels sont choisis dans le groupe de FeCl₃, CuCl₂, CuI₂, CuBr₂ et un complexe CuBr₂-sulfure de diméthyle, de préférence CuBr₂.
